(19) Europäisches Patentamt
European Patent Office
Office européen des brevets

(11) EP 4 599 674 A1

(12) EUROPEAN PATENT APPLICATION
published in accordance with Art. 153(4) EPC

(43) Date of publication:
**13.08.2025 Bulletin 2025/33**

(21) Application number: **24828719.5**

(22) Date of filing: **02.07.2024**

(51) International Patent Classification (IPC):
***A01N 47/24** (2006.01)* ***A01P 5/00** (2006.01)*
***C07C 315/02** (2006.01)* ***C07C 315/04** (2006.01)*
***C07C 317/44** (2006.01)*

(86) International application number:
**PCT/CN2024/103087**

(87) International publication number:
**WO 2025/129979 (26.06.2025 Gazette 2025/26)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **21.12.2023 CN 202311774550**

(71) Applicant: **Ningxia Hugerise Chemical Co., Ltd.**
**Shizuishan, Ningxia 753400 (CN)**

(72) Inventors:
- **Hao, Liyong**
  **Shizuishan, Ningxia 753400 (CN)**
- **Guo, Chuanlei**
  **Shizuishan, Ningxia 753400 (CN)**
- **Xu, Kun**
  **Shizuishan, Ningxia 753400 (CN)**
- **Yin, Cuiping**
  **Shizuishan, Ningxia 753400 (CN)**

(74) Representative: **Porta & Consulenti Associati S.p.A.**
**Via Winckelmann, 1**
**20146 Milano (IT)**

# (54) USE OF SULFOXAMYL, PREPARATION METHOD THEREFOR, AND OXIDATION SYSTEM

(57) Provided are use and a preparation method of sulfoxamyl, and an oxidation system, belonging to the technical field of pesticides. Provided is use of sulfoxamyl in killing nematodes. The sulfoxamyl shows an excellent killing activity against the nematodes as well as a desirable inhibitory effect on nematode eggs. Provided is an oxidation system for oxidizing a sulfur group in oxamyl and oxamyl oxime into a sulfone group. The oxidation system adopts hydrogen peroxide as an oxidant, which is safer, less explosive, and less expensive than peracetic acid; further, carboxylic acid or an aqueous solution of the carboxylic acid is used as a reaction medium, which is safer and more environmental-friendly than organic solvents. Moreover, the oxidation system can increase an oxidation rate and shorten a reaction time. The method for preparing the sulfoxamyl adopts hydrogen peroxide as an oxidant, which is safer and less expensive than peracetic acid; further, carboxylic acid or an aqueous solution of the carboxylic acid is used as a reaction medium, which is safer and more environmental-friendly than organic solvents. Moreover, the method shows the advantages of rapid oxidation, shortened reaction time, and high yield.

EP 4 599 674 A1

## Description

The present application claims priority to the Chinese Patent Application No. 202311774550.4, filed with the China National Intellectual Property Administration (CNIPA) on December 21, 2023, and entitled "USE AND PREPARATION METHOD OF SULFOXAMYL, AND OXIDATION SYSTEM", which is incorporated herein by reference in its entirety.

## TECHNICAL FIELD

The present disclosure relates to the technical field of pesticides, and in particular to use and a preparation method of sulfoxamyl, and an oxidation system.

## BACKGROUND

Plant parasitic nematodes (hereinafter referred to as "nematodes") generally keep concealment, invisible, have a short life cycle, a large reproduction coefficient, a wide variety of species, and a wide distribution and transmission, and are difficult to identify damage. Nematodes are one of the main pests in crop cultivation, directly affecting an entire growth period of crops, including beans, cereals, potatoes, beets, sweet potatoes, bananas, coconuts, tobacco, as well as vegetables such as cucumbers, tomatoes, and ginger. Globally crop yields are lost by not less than 12% annually due to nematode infection, leading to direct economic losses as high as $150 billion. With global climate change, crop system reform, and the rapid development of high-value agriculture, plant nematode diseases in China are becoming more serious and have become the second largest plant disease. Among them, root-knot nematodes and cyst nematodes are the two most serious crop pathogenic nematodes, and China suffers about $3.5 billion in losses each year due to the nematodes. The endoparasitic nature of nematodes requires them to complete most of their life cycle within a host plant, making them difficult to control since they are protected by tissues of the host plant.

The application of nematicides is an effective control measure for nematode pests. At present, there is generally high toxicity of commercialized nematicides in China, such as aldicarb, carbofuran, ethoprophos, fenamiphos, ethoprophos, avermectin, and fosthiazate. Except for the fosthiazate, which is of medium toxicity, all other varieties are of high toxicity. The long-term use of these highly toxic nematicides has caused serious damage to the environment, brought safety risks to production, storage, transportation, and applicators, and also posed serious hidden dangers to food safety. Therefore, most of the above nematicides have been banned in China. Currently, there are only a few varieties of nematicides active ingredients are registered in China, such as fosthiazate, dazomet, avermectin, and fluopyram. In this case, when nematodes become resistant to one of the nematicides, there are fewer alternatives to choose from, which to some extent causes a shortage of pesticides. Accordingly, it will remain a key to nematode disease control for a long time to come by finding novel, green, and highly-effective nematicides. Oxamyl, a commercially available excellent nematicide developed by DuPont in the United States in the 1970s, has been sold to most countries and regions in the world, including the United States, and also has been favored by the market for excellent insecticidal and nematicidal effects for over fifty years. However, the high toxicity of oxamyl prevents it from being authorized for registration in China, thus limiting its promotion and application in China.

Oxamyl is an oxime carbamate compound containing a methylthio group, with a chemical name of *N,N*-dimethyl-2-[[[(methylamino)carbonyl]oxy]imino]-2-(methylthio)acetamide, and has a structure shown in Formula I:

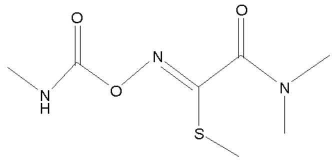

Formula I.

German patent Ger. Offen 2119700 has disclosed a sulfone compound corresponding to the compound shown in Formula I, namely *N,N*-dimethyl-2-[[[(methylamino)carbonyl]oxy]imino]-2-(methylsulfonyl)acetamide (sulfoxamyl), with a structure shown in Formula II:

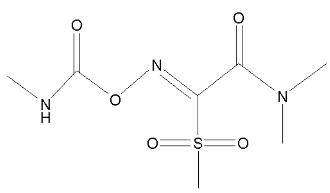

Formula II.

The German patent Ger. Offen 2119700 has only disclosed a fungicidal activity of the sulfoxamyl and a composition thereof, specifically studying an effect of the sulfoxamyl in preventing diseases of crops and ornamental plants caused by fungi of the class Phycomycetes, and the sulfoxamyl has not been commercially applied to date. It is worth noting that the patent does not study and mention a nematicidal activity and use of the sulfoxamyl.

The above German patent has also disclosed two methods for preparing sulfoxamyl. A first method is direct oxidation by using oxamyl as a raw material, based on a reaction formula as follows:

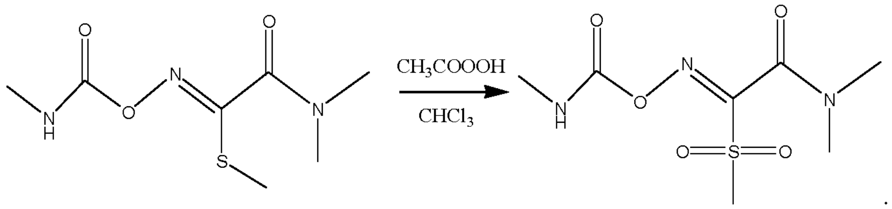


A second method is oxidation-condensation by using *N,N*-dimethyl-2-[hydroxy(imino)]-2-(methylthio)acetamide (hereinafter referred to as "oxamyl oxime") as a raw material. Specifically, the oxamyl oxime is oxidized to obtain *N,N*-dimethyl-2-[hydroxy(imino)]-2-(methylsulfonyl)acetamide (hereinafter referred to as "sulfoxamyl oxime"), which is then condensed with methyl isocyanate (MIC) or carbamoyl chloride (MCC) to obtain the sulfoxamyl, with a reaction formula is as follows:

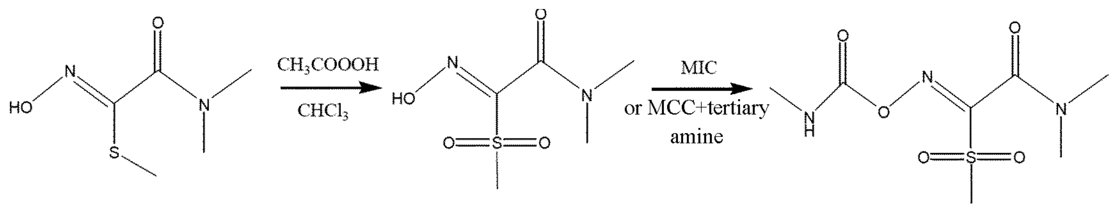


For the above two preparation methods, chloroform is used as solvent in the reaction of oxidizing methylthio group into methylsulfonyl group in the structural formula, and peracetic acid is used as the oxidant for oxidation, followed by conducting evaporation and concentration. An obtained crude product is washed with ether and then recrystallized with isopropanol as a solvent to obtain the corresponding target product. The existing methods have the following disadvantages and shortcomings: multiple organic solvents such as chloroform, diethyl ether, and isopropanol as well as highly dangerous peracetic acid as an oxidant pose serious explosion and fire safety hazards. Moreover, these toxic and hazardous substances also bring high occupational health risks. The raw material peracetic acid has the disadvantages of shortage of supply and high cost, limiting the large-scale production and application. A poor reaction rate takes not less than 12 h to complete the conversion.

## SUMMARY

In view of this, an object of the present disclosure is to provide use and a preparation method of sulfoxamyl, and an oxidation system. The sulfoxamyl shows an excellent killing activity against the nematodes as well as a desirable inhibitory effect on nematode eggs. The oxidation system does not use organic solvents or explosive oxidants and has a low cost.

To achieve the above object, the present disclosure provides the following technical solutions:
The present disclosure provides use of sulfoxamyl in killing nematodes.

The present disclosure provides an oxidation system for oxidizing a sulfur group in oxamyl or oxamyl oxime into a sulfone group, including an oxidant, a catalyst, and a reaction medium; where

the oxidant is hydrogen peroxide;
the catalyst is a strong acid, and
the reaction medium is selected from the group consisting of a carboxylic acid and an aqueous solution of the carboxylic acid.

In some embodiments, the strong acid is selected from the group consisting of an inorganic strong acid and an organic strong acid;

the inorganic strong acid is at least one selected from the group consisting of sulfuric acid, hydrochloric acid, nitric acid, perchloric acid, hydrobromic acid, hydroiodic acid, and phosphoric acid;

the organic strong acid is selected from the group consisting of a halogenated carboxylic acid and a sulfonic acid; the halogenated carboxylic acid is at least one selected from the group consisting of trifluoroacetic acid, trifluoropropionic acid, trifluorobutyric acid, monochloroacetic acid, dichloroacetic acid, and trichloroacetic acid;
the sulfonic acid is selected from the group consisting of benzenesulfonic acid and alkylbenzenesulfonic acid; and an alkyl group in the alkylbenzenesulfonic acid has a carbon number of 1 to 12.

In some embodiments, the reaction medium is at least one selected from the group consisting of formic acid, an aqueous solution of the formic acid, acetic acid, an aqueous solution of the acetic acid, propionic acid, an aqueous solution of the propionic acid, butyric acid, and an aqueous solution of the butyric acid.
The present disclosure further provides a method for preparing sulfoxamyl, including the following steps: subjecting oxamyl to oxidation in the presence of the oxidation system as described above to obtain the sulfoxamyl.
In some embodiments, a molar amount of the oxidant is not less than 2 times a molar amount of the oxamyl based on the hydrogen peroxide as an active ingredient;

a molar amount of the catalyst is not less than 0.01 times a molar amount of the oxamyl based on the strong acid as an active ingredient;
a mass of the reaction medium is not less than 2 times a mass of the oxamyl based on the carboxylic acid as an active ingredient; and
the oxidation is conducted at a temperature of 0 °C to 100 °C for 1 h to 24 h.

In some embodiments, after the oxidation is completed, the method further includes: cooling a resulting oxidation system to 0 °C, subjecting a resulting cooled product to solid-liquid separation to obtain a solid, and then drying the solid.
The present disclosure further provides another method for preparing sulfoxamyl, including the following steps:

subjecting oxamyl oxime to oxidation in the presence of the oxidation system as described above to obtain sulfoxamyl oxime; and
subjecting the sulfoxamyl oxime and a monomer to condensation to obtain the sulfoxamyl;
where the monomer is selected from the group consisting of methyl isocyanate (MIC) and carbamoyl chloride (MCC).

In some embodiments, a molar amount of the oxidant is not less than 2 times a molar amount of the oxamyl oxime based on the hydrogen peroxide as an active ingredient;

a molar amount of the catalyst is not less than 0.01 times a molar amount of the oxamyl oxime based on the strong acid as an active ingredient;
a mass of the reaction medium is not less than 2 times a mass of the oxamyl oxime based on the carboxylic acid as an active ingredient; and
the oxidation is conducted at a temperature of 0 °C to 100 °C for 1 h to 24 h.

In some embodiments, after the oxidation is completed, the method further includes: cooling a resulting oxidation system to 0 °C, subjecting a resulting cooled product to solid-liquid separation to obtain a solid, and then drying the solid.
The present disclosure provides use of sulfoxamyl in killing nematodes. The sulfoxamyl shows an excellent killing activity against the nematodes as well as a desirable inhibitory effect on nematode eggs.
The "sulfoxamyl" is the common name for the compound corresponding to the structural formula II. The inventor has applied to the National Pesticide Standardization Technical Committee and has been approved, such that the name of the compound II in the present disclosure is always referred to as "sulfoxamyl".
The present disclosure further provides an oxidation system for oxidizing a sulfur group in oxamyl or oxamyl oxime into a sulfone group, including an oxidant, a catalyst, and a reaction medium; where the oxidant is hydrogen peroxide, the catalyst is a strong acid, and the reaction medium is selected from the group consisting of a carboxylic acid and an aqueous solution of the carboxylic acid. The oxidation system adopts hydrogen peroxide as an oxidant, which is safer, less explosive, and less expensive than peracetic acid; further carboxylic acid or an aqueous solution of the carboxylic acid is used as a reaction medium, which is safer and more environmental-friendly than organic solvents. Moreover, the oxidation system can increase an oxidation rate and shorten a reaction time.
The present disclosure further provides a method for preparing sulfoxamyl, including the following steps: subjecting oxamyl to oxidation in the presence of the oxidation system as described above to obtain the sulfoxamyl. The method adopts hydrogen peroxide as an oxidant, which is safer, less explosive, and less expensive than peracetic acid; further carboxylic acid or an aqueous solution of the carboxylic acid is used as a reaction medium, which is safer and more environmental-friendly than organic solvents. Moreover, the method shows the advantages of fast oxidation, shortened reaction time, and high yield.

The present disclosure further provides another method for preparing sulfoxamyl, including the following steps: subjecting oxamyl oxime to oxidation in the presence of the oxidation system as described above to obtain sulfoxamyl oxime; and subjecting the sulfoxamyl oxime and a monomer to condensation to obtain the sulfoxamyl; where the monomer is selected from the group consisting of MIC and MCC. The method adopts hydrogen peroxide as an oxidant, which is safer, less explosive, and less expensive than peracetic acid; futher carboxylic acid or an aqueous solution of the carboxylic acid is used as a reaction medium, which is safer and more environmental-friendly than organic solvents. Moreover, the method shows the advantages of fast oxidation, shortened reaction time, and high yield.

Furthermore, after the oxidation is completed, the method further includes: cooling a resulting oxidation system to 0 °C, subjecting a resulting cooled product to solid-liquid separation to obtain a solid, and then drying the solid. Compared with the post-treatment in the German patent which requires unit operations such as solvent evaporation, ether washing, and isopropanol recrystallization, the method disclosed in the present disclosure has fewer post-treatment steps and a simple process.

## DETAILED DESCRIPTION OF THE EMBODIMENTS

The present disclosure provides use of sulfoxamyl in killing nematodes.

In the present disclosure, the sulfoxamyl has a structure shown in Formula II:

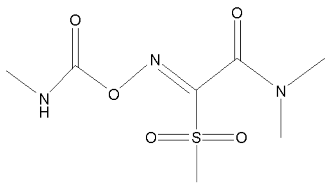

Formula II.

In some embodiments of the present disclosure, under a condition that the sulfoxamyl is used to kill nematodes, it is used in the form of a composition; where a dosage form of the composition is at least one selected from the group consisting of a granule, a powder, a wettable powder, an emulsifiable concentrate, a suspension concentrate, a dry suspension, a concentrated emulsion, and a sustained-release agent.

In some embodiments of the present disclosure, an active ingredient for the composition includes the sulfoxamyl. In some embodiments of the present disclosure, the active ingredient for the composition further includes other active ingredients that can be used in combination with the sulfoxamyl.

In some embodiments of the present disclosure, the composition further includes at least one selected from the group consisting of an auxiliary agent, a carrier, and a solvent. There is no specific limitation on amounts for the auxiliary agent, carrier, and solvent, and those skilled in the art can set them according to conventional technical means.

In some embodiments of the present disclosure, the auxiliary agent includes at least one selected from the group consisting of a binder and a stabilizer. In some embodiments of the present disclosure, the binder includes at least one selected from the group consisting of paraffin and PVA. In some embodiments of the present disclosure, the stabilizer is at least one selected from the group consisting of phosphoric acid, oxalic acid, tartaric acid, and citric acid.

In some embodiments of the present disclosure, the carrier is at least one selected from the group consisting of quartz sand, attapulgite, and a clay particle.

In some embodiments of the present disclosure, the solvent is at least one selected from the group consisting of water, an alcohol, an ester, an aromatic hydrocarbon, and an ether.

In some embodiments of the present disclosure, a method for field application of the composition is selected from the group consisting of hole application, furrow application, broadcast application, blending with a toxic soil, and root irrigation.

The present disclosure provides an oxidation system for oxidizing a sulfur group in oxamyl and oxamyl oxime into a sulfone group, including an oxidant, a catalyst, and a reaction medium; where

the oxidant is hydrogen peroxide;
the catalyst is a strong acid;
and the reaction medium is selected from the group consisting of a carboxylic acid and an aqueous solution of the carboxylic acid.

In the present disclosure, the oxidation system includes an oxidant, and the oxidant is hydrogen peroxide. In some embodiments of the present disclosure, the hydrogen peroxide has a mass concentration not greater than 75%, preferably 27%.

In the present disclosure, the oxidation system includes a catalyst, and the catalyst is a strong acid. In some

embodiments of the present disclosure, the strong acid is selected from the group consisting of an inorganic strong acid and an organic strong acid, preferably the inorganic strong acid. In some embodiments of the present disclosure, the inorganic strong acid is at least one selected from the group consisting of sulfuric acid, hydrochloric acid, nitric acid, perchloric acid, hydrobromic acid, hydroiodic acid, and phosphoric acid, preferably the sulfuric acid. In some embodiments of the present disclosure, the sulfuric acid has a mass concentration of 98%. In some embodiments of the present disclosure, the organic strong acid is selected from the group consisting of a halogenated carboxylic acid and a sulfonic acid. In some embodiments of the present disclosure, the halogenated carboxylic acid is at least one selected from the group consisting of trifluoroacetic acid, trifluoropropionic acid, trifluorobutyric acid, monochloroacetic acid, dichloroacetic acid, and trichloroacetic acid. In some embodiments of the present disclosure, the sulfonic acid is selected from the group consisting of benzenesulfonic acid and alkylbenzenesulfonic acid; where an alkyl group in the alkylbenzenesulfonic acid has a carbon number of 1 to 12.

In the present disclosure, the oxidation system includes a reaction medium, where the reaction medium is selected from the group consisting of a carboxylic acid and an aqueous solution of the carboxylic acid. In some embodiments of the present disclosure, the reaction medium is at least one selected from the group consisting of formic acid, an aqueous solution of the formic acid, acetic acid, an aqueous solution of the acetic acid, propionic acid, an aqueous solution of the propionic acid, butyric acid, and an aqueous solution of the butyric acid. In some embodiments of the present disclosure, the aqueous solution of the carboxylic acid has a mass concentration not less than 50%. In some embodiments of the present disclosure, the carboxylic acid is at least one selected from the group consisting of formic acid, acetic acid, propionic acid, and butyric acid, preferably the acetic acid.

In some embodiments of the present disclosure, the amounts for the oxidant, catalyst, and reaction medium in the oxidation system are described in detail in the specific process for preparing sulfoxamyl.

The present disclosure further provides a method for preparing sulfoxamyl, including the following steps: subjecting oxamyl to oxidation in the presence of the oxidation system to obtain the sulfoxamyl.

In the present disclosure, the oxamyl has a structure shown in Formula I:

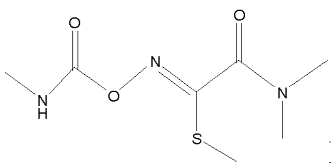

Formula I.

In some embodiments of the present disclosure, a molar amount of the oxidant is not less than 2 times, preferably 2.5 to 3 times a molar amount of the oxamyl based on the hydrogen peroxide as an active ingredient.

In some embodiments of the present disclosure, a molar amount of the catalyst is not less than 0.01 times, preferably 0.3 to 0.6 times a molar amount of the oxamyl based on the strong acid as an active ingredient.

In some embodiments of the present disclosure, a mass of the reaction medium is not less than 2 times, more preferably 2 to 3 times a mass of the oxamyl based on the carboxylic acid as an active ingredient. Specifically, for the aqueous solution of the carboxylic acid, a mass of the carboxylic acid in the aqueous solution of the carboxylic acid is not less than 2 times a mass of the oxamyl.

In some embodiments of the present disclosure, the oxidation is conducted at a temperature of 0 °C to 100°C, preferably a temperature of 35 °C to 50 °C for 1 h to 24 h.

In some embodiments of the present disclosure, the oxidation of oxamyl under the action of the oxidation system includes the following steps: subjecting the oxamyl and the reaction medium to first stirring and mixing, adding the catalyst and the oxidant sequentially and subjecting a resulting mixture to second stirring and mixing, and then conducting the oxidation. There is no specific limitation on rotation speed and time for the first stirring and mixing, as long as the oxamyl can be completely dissolved in the reaction medium. In some embodiments of the present disclosure, the second mixing and stirring is conducted at room temperature for 10 min.

In some embodiments of the present disclosure, after the oxidation is completed, the method further includes: cooling a resulting oxidation system to 0 °C, subjecting a resulting cooled product to solid-liquid separation to obtain a solid, and then drying the solid. In some embodiments of the present disclosure, the drying is conducted at 60 °C.

In the present disclosure, a reaction formula for preparing the sulfoxamyl by oxidation of the oxamyl in the oxidation system is as follows:

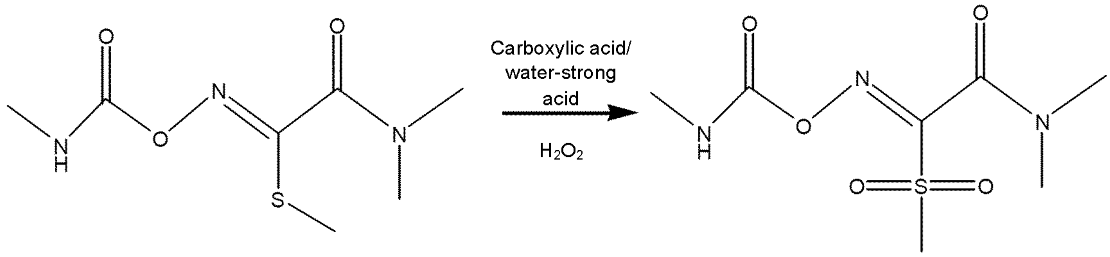


.

The present disclosure further provides another method for preparing sulfoxamyl, including the following steps:

subjecting oxamyl oxime to oxidation in the presence of the oxidation system to obtain sulfoxamyl oxime; and
subjecting the sulfoxamyl oxime and a monomer to condensation to obtain the sulfoxamyl;
where the monomer is selected from the group consisting of methyl isocyanate (MIC) and carbamoyl chloride (MCC).

In the present application, oxamyl oxime is subjected to oxidation in the presence of the oxidation system to obtain sulfoxamyl oxime.
In some embodiments of the present disclosure, a molar amount of the oxidant is not less than 2 times, preferably 2.5 to 3 times a molar amount of the oxamyl oxime based on the hydrogen peroxide as an active ingredient.
In some embodiments of the present disclosure, a molar amount of the catalyst is not less than 0.01 times, preferably 0.3 to 0.6 times a molar amount of the oxamyl oxime based on the strong acid as an active ingredient.
In some embodiments of the present disclosure, a mass of the reaction medium is not less than 2 times, preferably 2 to 3 times a mass of the oxamyl oxime based on the carboxylic acid as an active ingredient.
In some embodiments of the present disclosure, the oxidation is conducted at a temperature of 0 °C to 100 °C, preferably a temperature of 35 °C to 50 °C for 1 h to 24 h.
In some embodiments of the present disclosure, the oxidation of oxamyl oxime under the action of the oxidation system includes the following steps: subjecting the oxamyl oxime and the reaction medium to first stirring and mixing, adding the catalyst and the oxidant sequentially and subjecting a resulting mixture to second stirring and mixing, and then conducting the oxidation. There is no specific limitation on rotation speed and time for the first stirring and mixing, as long as the oxamyl oxime can be completely dissolved in the reaction medium. In some embodiments of the present disclosure, the second mixing and stirring is conducted at room temperature for 10 min.
In some embodiments of the present disclosure, after the oxidation is completed, the method further includes: cooling a resulting oxidation system to 0 °C, subjecting a resulting cooled product to solid-liquid separation to obtain a solid, and then drying the solid. In some embodiments of the present disclosure, the drying is conducted at 60 °C.
In the present disclosure, a reaction formula for preparing the sulfoxamyl oxime by oxidation of the oxamyl oxime in the oxidation system is follows:

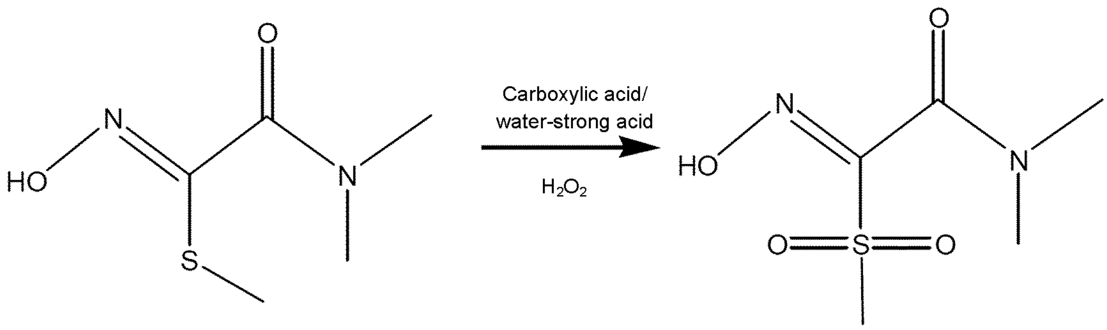


.

In the present disclosure, the sulfoxamyl oxime and a monomer are subjected to condensation to obtain the sulfoxamyl, with a reaction formula as follows:

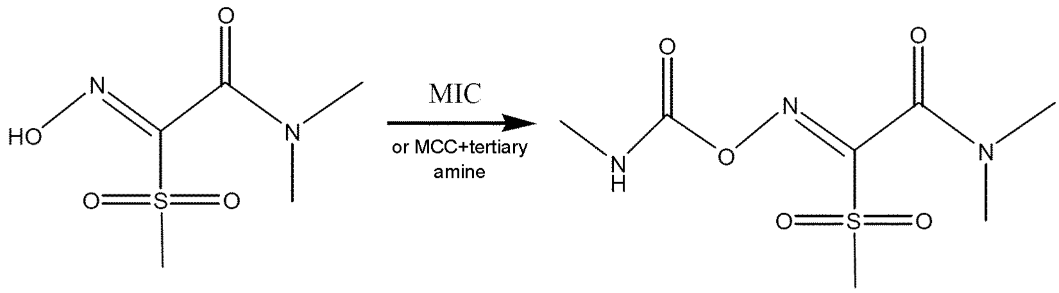


In the present disclosure, the monomer is selected from the group consisting of MIC and MCC.

In some embodiments of the present disclosure, under a condition that the monomer is the MIC, a molar ratio of the sulfoxamyl oxime to the MIC is in a range of 1:(1.0-1.5).

In some embodiments of the present disclosure, under a condition that the monomer is the MCC, the method further includes: adding an acid-binding agent; where the acid-binding agent includes an organic tertiary amine; and the organic tertiary amine includes at least one selected from the group consisting of TEA and pyridine. In some embodiments of the present disclosure, a molar ratio of the sulfoxamyl oxime, the MCC, and the acid-binding agent is in a range of 1:(1.0-1.8):(1.0-1.8).

In some embodiments of the present disclosure, a reaction medium for the condensation includes at least one selected from the group consisting of toluene, water, dichloromethane (DCM), dichloroethane (DCE), and ethyl acetate.

In some embodiments of the present disclosure, the condensation is conducted at a temperature of 10 °C to 80 °C, preferably a temperature of 20 °C to 60 °C for 0.5 h to 2 h.

In some embodiments of the present disclosure, under a condition that the monomer is the MIC, after the condensation is completed, the method further includes: cooling a resulting condensation feed liquid, conducting solid-liquid separation, and then drying a resulting filter residue.

In some embodiments of the present disclosure, under a condition that the monomer is the MCC, after the condensation is completed, the method further includes: adding a water-soluble salt into a resulting condensation feed liquid, conducting solid-liquid separation, and then drying a resulting filter residue.

The use and the preparation method of sulfoxamyl, and the oxidation system provided by the present disclosure will be described in detail below with reference to the examples, but they should not be construed as limiting the scope of the present disclosure.

1. Use of sulfoxamyl as an active substance in killing nematodes was provided, and its safety and effectiveness were proved by toxicology test, hydrolysis test, and efficacy test.

1.1 Safety

Safety is reflected in two aspects. First is the acute toxicity safety to mammals. The sulfoxamyl has been confirmed to be low toxic in toxicity classification through acute oral and acute percutaneous toxicology tests. Second, the half-life is short. The sulfoxamyl is easy to decompose under neutral conditions, with a half-life of only 2 h, and is completely decomposed in about 8 h, indicating that there are relatively safe low-level pesticide residues for crops and the environment.

1.1.1 Toxicology test

1.1.1.1 Acute oral toxicity test, conclusion: low toxicity

Materials: the test agent 98% sulfoxamyl technical, batch number 20230708-1, was provided by Ningxia Hugerise Chemical Co., Ltd. Experimental animals: SPF-grade SD rats, half male and half female, weighing 180 g to 220 g, were provided by Jinan Pengyue Experimental Animal Breeding Co., Ltd., license number SCXK (Lu) 20190003. Rearing environment: the rats were housed in an SPF-grade animal room with free access to water and food, a temperature of 20 °C to 25 °C, a humidity of 40% to 60%, and a 12 h/12 h light cycle. Feed: conventional pellet feed was provided by Jinan Pengyue Experimental Animal Breeding Co., Ltd.

The test was conducted in accordance with "Toxicological test methods of pesticides for registration, GB15670.5-2017", "Acute toxicity test, GB15193.3-2003" and relevant acute toxicity research data.

Methods: a single oral fixed-dose procedure was conducted to preliminarily explore the toxic dose range of sulfoxamyl. $LD_{50}$ and confidence limits were calculated by using the probability unit method (Bliss method). SD rats were

randomly divided into 5 groups, 10 rats in each group, half of them were male and half were female. The dosages administered to each group were 474.61 mg/kg, 632.81 mg/kg, 843.75 mg/kg, 1,125 mg/kg, and 1,500 mg/kg. The common ratio value among each dose group was 0.75. After a single oral administration, the mortality rate of the experimental animals was recorded within 24 h and 14 days after administration, while the general condition of the animals was observed, and the food intake and body weight changes were recorded.

1) The results of the fixed-dose procedure are shown in Table 1. It is seen from Table 1 that no animals die in the 50 mg/kg and 500 mg/kg groups (6 animals in each group, half male and half female); 8 (4 males and 4 females) of the 10 animals (half male and half female) in the 1,000 mg/kg group die within 24 h; all animals in the 2,000 mg/kg group (a total of 6 animals, half males and half females) die. This suggests that the lethal dose range of the drug is approximately 500 mg/kg to 2,000 mg/kg.

**Table 1 Toxicity test results of sulfoxamyl and oxamyl (single oral fixed-dose procedure)**

| Sample name | Dose/mg/kg | Death count | Main clinical symptom |
|---|---|---|---|
| Sulfoxamyl | 50 | 0/6 | Asymptomatic |
| | 500 | 0/6 | Some rats with intense abdominal breathing, squinting eyes, listlessness, and runny nose and mouth |
| | 1000 | 8/10 | Intense abdominal breathing, malaise, drooling, tremors |
| | 2000 | 6/6 | Intense abdominal breathing, malaise, drooling, limb struggling |
| Oxamyl | 500 | 6/6 | All rats died within 5 min with intense struggling |

2) The results of the probability unit method (Bliss method) are shown in Table 2. It is seen from Table 2 that 24 h after administration, no experimental animals in the 474.61 mg/kg dose group die, 3 animals in the 632.81 mg/kg dose group (2 males and 1 female) die, 6 animals in the 843.75 mg/kg dose group (3 males and 3 females) die, 7 animals in the 1,125 mg/kg dose group (3 males and 4 females) die, and all animals in the 1,500 mg/kg dose group die.

**Table 2 Results of 24-h determination of lethal dose of sulfoxamyl (Bliss method)**

| Dose/mg/kg | Death count | Main clinical symptom |
|---|---|---|
| 474.61 | 0/10 | Asymptomatic |
| 632.81 | 3/10 | Intense abdominal breathing, squinting eyes, listlessness, and runny nose and mouth |
| 843.75 | 6/10 | Intense abdominal breathing, malaise, drooling, tremors |
| 1125 | 8/10 | Intense abdominal breathing, malaise, drooling, tremors |
| 1500 | 10/10 | Intense abdominal breathing, malaise, drooling, limb struggling |

3) There are no new deaths of rats 14 days after administration compared to 24 h later.

According to the above test dose, number of animals, number of deaths and other data, SPSS statistical software was used to calculate $LD_{50}$ by Probit regression, and the results are shown in Table 3. Table 3 shows that the $LD_{50}$ value of sulfoxamyl is 821.88 mg/kg, and its 95% confidence limit is 695.55 mg/kg to 964.83 mg/kg.

**Table 3 Confidence limits**

| Probit | 95% confidence limits for dose | | | 95% confidence limits for log(dose) | | |
|---|---|---|---|---|---|---|
| Probability | Estimation | Lower limit | Upper limit | Estimation | Lower limit | Upper limit |
| 0.010 | 379.474 | 188.027 | 500.163 | 2.579 | 2.274 | 2.699 |
| 0.020 | 415.442 | 221.740 | 533.944 | 2.619 | 2.346 | 2.727 |
| 0.030 | 440.010 | 246.088 | 556.800 | 2.643 | 2.391 | 2.746 |
| 0.040 | 459.445 | 266.074 | 574.803 | 2.662 | 2.425 | 2.760 |
| 0.050 | 475.885 | 283.460 | 590.005 | 2.678 | 2.452 | 2.771 |

(continued)

| Probit | 95% confidence limits for dose | | | 95% confidence limits for log(dose) | | |
|---|---|---|---|---|---|---|
| Probability | Estimation | Lower limit | Upper limit | Estimation | Lower limit | Upper limit |
| 0.060 | 490.341 | 299.095 | 603.370 | 2.690 | 2.476 | 2.781 |
| 0.070 | 503.377 | 313.464 | 615.435 | 2.702 | 2.496 | 2.789 |
| 0.080 | 515.342 | 326.868 | 626.529 | 2.712 | 2.514 | 2.797 |
| 0.090 | 526.471 | 339.512 | 636.874 | 2.721 | 2.531 | 2.804 |
| 0.100 | 536.928 | 351.541 | 646.624 | 2.730 | 2.546 | 2.811 |
| 0.150 | 582.478 | 405.444 | 689.639 | 2.765 | 2.608 | 2.839 |
| 0.200 | 621.421 | 453.080 | 727.523 | 2.793 | 2.656 | 2.862 |
| 0.250 | 656.898 | 497.238 | 763.434 | 2.817 | 2.697 | 2.883 |
| 0.300 | 690.481 | 539.228 | 799.144 | 2.839 | 2.732 | 2.903 |
| 0.350 | 723.131 | 579.741 | 835.962 | 2.859 | 2.763 | 2.922 |
| 0.400 | 755.539 | 619.159 | 875.053 | 2.878 | 2.792 | 2.942 |
| 0.450 | 788.276 | 657.707 | 917.587 | 2.897 | 2.818 | 2.963 |
| 0.500 | 821.879 | 695.552 | 964.829 | 2.915 | 2.842 | 2.984 |
| 0.550 | 856.914 | 732.896 | 1018.211 | 2.933 | 2.865 | 3.008 |
| 0.600 | 894.044 | 770.054 | 1079.448 | 2.951 | 2.887 | 3.033 |
| 0.650 | 934.112 | 807.526 | 1150.753 | 2.970 | 2.907 | 3.061 |
| 0.700 | 978.282 | 846.077 | 1235.241 | 2.990 | 2.927 | 3.092 |
| 0.750 | 1028.295 | 886.864 | 1337.725 | 3.012 | 2.948 | 3.126 |
| 0.800 | 1087.001 | 931.713 | 1466.410 | 3.036 | 2.969 | 3.166 |
| 0.850 | 1159.674 | 983.855 | 1637.102 | 3.064 | 2.993 | 3.214 |
| 0.900 | 1258.055 | 1050.184 | 1886.539 | 3.100 | 3.021 | 3.276 |
| Conclusion: the acute oral toxicity $LD_{50}$ value of sulfoxamyl is 821.88 mg/kg. According to the national standard GB15670-1995 "Toxicological test methods of pesticides for registration", the toxicity level is low. | | | | | | |

1.1.1.2 Acute skin toxicity test, conclusion: low toxicity

Materials: the test agent 98% sulfoxamyl technical, batch number 20230708-1, was provided by Ningxia Hugerise Chemical Co., Ltd. Experimental animals: SPF-grade SD rats, half male and half female, weighing 180 g to 220 g, were provided by Jinan Pengyue Experimental Animal Breeding Co., Ltd., license number SCXK (Lu) 20190003. Rearing environment: the rats were housed in an SPF-grade animal room with free access to water and food, a temperature of 20 °C to 25 °C, a humidity of 40% to 60%, and a 12 h/12 h light cycle. Feed: conventional pellet feed was provided by Jinan Pengyue Experimental Animal Breeding Co., Ltd.

The test was conducted in accordance with "Toxicological test methods of pesticides for registration, GB15670.5-2017", "Acute toxicity test, GB15193.3-2003" and relevant acute toxicity research data.

Method: the sulfoxamyl was ground into powder, passed through a 200-mesh sieve, and fully moistened with 0.5% tragacanth solution to ensure desirable contact between the drug and the skin. The dose limit of the drug's transdermal toxicity was preliminarily determined through a limit test. According to the "Toxicological test methods of pesticides for registration, GB/T 15670.5-2017", 2,000 mg/kg was selected as the dose limit. 10 SD rats were selected, half male and half female. After removing the fur and preparing the skin on the midline of the rats' backs, the prepared skin area was 36 $cm^2$ to 40 $cm^2$. The drug was evenly applied to the fur-removed area and covered and fixed with multiple layers of sterile gauze; after 24 h of exposure to the poison, the residual drug on the skin was removed; the rats were observed once a day for 14 consecutive days, and their poisoning reaction, development process, and death were recorded.

Results: none of the 10 rats die, and their skin is intact, normal in color, and without redness or swelling. The rats have no other systemic lesions. Sulfoxamyl at 2,000 mg/kg has no percutaneous toxicity, and the results are shown in

Table 4.

**Table 4 Results of acute skin toxicity test of sulfoxamyl**

| SN | Gender | Body weight/g | Administration dose/mL | Administration area/$cm^2$ | Main clinical symptom |
|---|---|---|---|---|---|
| 1 | ♀ | 210.56 | 1.05 | 40 | Normal skin with no abnormalities |
| 2 | ♀ | 226.62 | 1.13 | 36 | |
| 3 | ♀ | 213.64 | 1.06 | 36 | |
| 4 | ♀ | 207.80 | 1.04 | 36 | |
| 5 | ♀ | 223.36 | 1.12 | 40 | |
| 6 | ♂ | 256.35 | 1.28 | 30 | |
| 7 | ♂ | 245.11 | 1.23 | 40 | |
| 8 | ♂ | 245.29 | 1.23 | 36 | |
| 9 | ♂ | 254.53 | 1.27 | 42 | |
| 10 | ♂ | 253.60 | 1.27 | 36 | |
| Conclusion: the acute percutaneous toxicity $LD_{50}$ value of sulfoxamyl is greater than 2,000 mg/kg. According to the national standard GB15670-1995 "Toxicological test methods of pesticides for registration", the toxicity level is low. | | | | | |

1.1.2 Half-life test

A 98% sulfoxamyl sample was quantitatively dissolved in neutral distilled water, and samples were taken and injected at certain time intervals under HPLC detection. Data were collected according to the area normalization method based on the integrated sulfoxamyl peak area ratio. The results are shown in Table 5.

**Table 5 Hydrolysis test results of sulfoxamyl (25 °C)**

| Time/h | Changes in ratio of sulfoxamyl peak area under HPLC/% |
|---|---|
| 0 | 99.86 |
| 0.5 | 73 |
| 1 | 63 |
| 2 | 48 |
| 3 | 32 |
| 4 | 20 |
| 5 | 11 |
| 6 | 5 |
| 7 | 4 |
| 8 | 3 |

Conclusion: the sulfoxamyl has a half-life of about 2 h in neutral aqueous solution and is an easily hydrolyzed substance, indicating that it decomposes quickly under humid conditions in the natural environment, suggesting a lower level of pesticide residues on crops and a low environmental safety risk.

1.2. In terms of effectiveness, the indoor efficacy test shows that sulfoxamyl has a high killing activity against southern root-knot nematode J2 and also has a desirable inhibitory effect on nematode eggs. The experimental process was as follows:

Materials: the tested nematodes were collected from three different areas, namely cucumber crops in Dawenkou Town, Tai'an City, Shandong Province, and were all identified as southern root-knot nematodes (*Meloidogyne incognita*). The diseased roots were washed, cut into small sections of 0.5 cm to 1 cm, rinsed in 1% NaClO solution for about 30 s to 60 s, and rinsed with 200-mesh and 500-mesh sieves to collect nematode eggs for later use. The collected eggs were incubated

at 25 °C using the Bayman funnel method and the second-instar larvae were collected 1 time per 24 h to ensure that the second-instar larvae selected each time were fresh and of consistent vitality.

The agent was 98% sulfoxamyl technical, and the comparison agents were 96% oxamyl technical and 87% fosthiazate technical.

The preparation for the agent was in accordance with the provisions for preparation of the liquid medicine in the Agricultural Industry Standard of the People's Republic of China NYT 1833.1-2009 "Guideline for laboratory bioassay of pesticides", as shown in Table 6.

**Table 6 Dosage setting of sulfoxamyl for toxicity test against** *Meloidogyne incognita*

| Group | Dose/mg/L |
|---|---|
| Oxamyl group | 400, 200, 100, 50, 25 |
| Sulfoxamyl group | 100, 50, 25, 12.5, 6.25, 3.1 |
| Fosthiazate group | 200, 100, 50, 25, 12.5, 6.25 |
| Blank control group | Mother liquor (no active agent) |

Methods: according to the experimental plan, the test agent was diluted to different mass concentrations, and 0.5 mL of the agent solution and 0.5 mL of the nematode solution (containing about 100 nematodes) were added into a 24-well cell culture plate. The treatment with each concentration of the agent was repeated 4 times, and auxiliary agent control and blank control were also conducted. After being treated in an incubator at 25 °C for 24 h, the nematodes were determined alive or dead by acupuncture under a stereomicroscope, the number of surviving nematodes was counted, and their mortality and corrected mortality were calculated.

$$Mortality\ (\%) = \frac{Number\ of\ died\ nematodes}{Total\ number\ of\ treated\ nematodes} \times 100$$

$$Corrected\ mortality\ (\%) = \frac{Mortality\ of\ treatment\ group - Mortality\ of\ control\ group}{100 - Mortality\ of\ control\ group} \times 100$$

At 24 h, 48 h, and 72 h after drug administration, the mortality of the auxiliary agent control treatment and the blank control treatment is less than 2%, which meets the bioassay requirements and is a valid experiment.

The results are shown in Tables 7 to 10.

**Table 7 Toxicity results of three pesticides against *Meloidogyne incognita* (24 h)**

| Agent name | $LC_{50}$ (mg/L) | $LC_{90}$ (mg/L) | Regression equation of virulence | $R^2$ |
|---|---|---|---|---|
| Oxamyl | 94.76 | 273.89 | Y=2.7804x-0.4959 | 0.9962 |
| Sulfoxamyl | 12.52 | 52.89 | Y=2.0479x+2.7523 | 0.9725 |
| Fosthiazate | 14.57 | 146.50 | Y=1.2786x+3.5124 | 0.9763 |

**Table 8 Toxicity results of two pesticides against *Meloidogyne incognita* (48 h)**

| Agent name | $LC_{50}$ (mg/L) | $LC_{90}$ (mg/L) | Regression equation of virulence | $R^2$ |
|---|---|---|---|---|
| Oxamyl | 64.41 | 181.92 | Y=2.8421x-0.413 | 0.9949 |
| Sulfoxamyl | 10.23 | 56.41 | Y=1.7289x+3.2538 | 0.9705 |

**Table 9 Toxicity results of two pesticides against *Meloidogyne incognita* (72 h)**

| Agent name | $LC_{50}$ (mg/L) | $LC_{90}$ (mg/L) | Regression equation of virulence | $R^2$ |
|---|---|---|---|---|
| Oxamyl | 49.61 | 131.82 | Y=3.0198x-0.1205 | 0.9982 |
| Sulfoxamyl | 7.41 | 36.84 | Y=1.8408x+3.3981 | 0.9342 |

**Table 10 Inhibitory effects of two pesticides on hatching of *Meloidogyne incognita* eggs (7 d)**

| Agent name | $IC_{50}$ (mg/L) | $IC_{90}$ (mg/L) | Regression equation of virulence | $R^2$ |
|---|---|---|---|---|
| Oxamyl | 91.6100 | 479.5988 | y=1.5027+1.7826x | 0.9662 |
| Sulfoxamyl | 59.4760 | 510.8371 | y=2.5653+1.3722x | 0.9715 |

As shown in Tables 7 to 9, the $LC_{50}$ values of sulfoxamyl are 12.52 mg/L, 10.23 mg/L, and 7.41 mg/L, respectively, 24 h, 48 h, and 72 h after treatment with *Meloidogyne incognita*. The three time periods are all lower than the $LC_{50}$ values of the selected control agent oxamyl, which are 94.76 mg/L, 64.41 mg/L, and 49.61 mg/L, indicating that sulfoxamyl has excellent indoor nematicidal activity, which is higher than that of oxamyl. As shown in Table 7, the $LC_{50}$ value of sulfoxamyl is 12.52 mg/L and the $LC_{90}$ value is 52.89 mg/L 24 h after the treatment of *Meloidogyne incognita*. The $LC_{50}$ value of the control agent fosthiazate is 14.57 mg/L and the $LC_{90}$ value is 146.50 mg/L, indicating that the indoor nematicidal activity of sulfoxamyl is higher than that of fosthiazate. As shown in Table 10, the sulfoxamyl has a desirable inhibitory effect on the hatching of *Meloidogyne incognita* eggs.

Specific data are shown in Tables 11 to 14.

**Table 11 Toxicity test data for control agent oxamyl against *Meloidogyne incognita***

| Oxamyl | | | 24 h | | | 48 h | | | 72 h | | |
|---|---|---|---|---|---|---|---|---|---|---|---|
| | Repetition | Total | Death count | Mortality (%) | Corrected mortality (%) | Death count | Mortality (%) | Corrected mortality (%) | Death count | Mortality (%) | Corrected mortaliy (%) |
| CK | 1 | 131 | 1 | 0.76 | | 1 | 0.76 | | 3 | 2.29 | |
| | 2 | 118 | 2 | 1.69 | | 3 | 2.54 | | 3 | 2.54 | |
| | 3 | 125 | 1 | 0.80 | | 1 | 0.80 | | 1 | 0.80 | |
| | 4 | 114 | 2 | 1.75 | | 2 | 1.75 | | 2 | 1.75 | |
| Average | | 122 | 1.5 | 1.23 | | 2 | 1.43 | | 2 | 1.84 | |
| 25 | 1 | 112 | 9 | 8.04 | 6.89 | 15 | 13.39 | 12.13 | 20 | 17.86 | 16.31 |
| | 2 | 101 | 4 | 3.96 | 2.76 | 18 | 17.82 | 16.63 | 25 | 24.75 | 23.34 |
| | 3 | 109 | 5 | 4.59 | 3.40 | 9 | 8.26 | 6.92 | 19 | 17.43 | 15.88 |
| | 4 | 124 | 6 | 4.84 | 3.65 | 10 | 8.06 | 6.73 | 21 | 16.94 | 15.37 |
| Average | | 111.5 | 6 | 5.38 | 4.18 | 13 | 11.66 | 10.60 | 21 | 19.06 | 17.73 |
| 50 | 1 | 101 | 24 | 23.76 | 22.81 | 30 | 29.70 | 28.68 | 41 | 40.59 | 39.48 |
| | 2 | 134 | 41 | 30.60 | 29.73 | 61 | 45.52 | 44.73 | 76 | 56.72 | 55.90 |
| | 3 | 108 | 30 | 27.78 | 26.88 | 44 | 40.74 | 39.88 | 65 | 60.19 | 59.44 |
| | 4 | 106 | 19 | 17.92 | 16.90 | 34 | 32.08 | 31.09 | 49 | 46.23 | 45.22 |
| Average | | 112.25 | 28.5 | 25.39 | 24.08 | 42 | 37.64 | 36.09 | 58 | 51.45 | 50.01 |
| 100 | 1 | 127 | 74 | 58.27 | 57.75 | 90 | 70.87 | 70.44 | 97 | 76.38 | 75.93 |
| | 2 | 109 | 54 | 49.54 | 48.91 | 84 | 77.06 | 76.73 | 89 | 81.65 | 81.31 |
| | 3 | 120 | 71 | 59.17 | 58.66 | 92 | 76.67 | 76.33 | 101 | 84.17 | 83.87 |
| | 4 | 112 | 68 | 60.71 | 60.23 | 91 | 81.25 | 80.98 | 109 | 97.32 | 97.27 |
| Average | | 117 | 66.75 | 57.05 | 56.39 | 89 | 76.28 | 76.12 | 99 | 84.62 | 84.60 |
| 200 | 1 | 111 | 78 | 70.27 | 69.90 | 104 | 93.69 | 93.60 | 108 | 97.30 | 97.25 |
| | 2 | 104 | 90 | 86.54 | 86.37 | 94 | 90.38 | 90.24 | 98 | 94.23 | 94.12 |
| | 3 | 105 | 89 | 84.76 | 84.57 | 97 | 92.38 | 92.27 | 99 | 94.29 | 94.18 |
| | 4 | 95 | 90 | 94.74 | 94.67 | 93 | 97.89 | 97.86 | 94 | 98.95 | 98.93 |

(continued)

| Oxamyl | | | 24 h | | | 48 h | | | 72 h | | |
|---|---|---|---|---|---|---|---|---|---|---|---|
| | Repe tition | Total | Death count | Mortality (%) | Corrected mortality (%) | Death count | Mortalit y (%) | Correct ed mortality (%) | Death count | Mortalit y (%) | Correcte d mortaliy (%) |
| Average | | 103.75 | 86.75 | 83.61 | 83.88 | 97 | 93.49 | 93.50 | 100 | 96.14 | 96.12 |
| 400 | 1 | 103 | 99 | 96.12 | 96.07 | 101 | 98.06 | 98.03 | 103 | 100.00 | 100.00 |
| | 2 | 111 | 101 | 90.99 | 90.88 | 108 | 97.30 | 97.26 | 110 | 99.10 | 99.08 |
| | 3 | 121 | 117 | 96.69 | 96.65 | 119 | 98.35 | 98.32 | 120 | 99.17 | 99.16 |
| | 4 | 118 | 112 | 94.92 | 94.85 | 117 | 99.15 | 99.14 | 118 | 100.00 | 100.00 |
| Average | | 113.25 | 107.25 | 94.70 | 94.61 | 111 | 98.23 | 98.19 | 113 | 99.56 | 99.56 |

**Table 12 Toxicity test data for control agent fosthiazate against *Meloidogyne incognita***

| Fosthiazate | | | 24 h | | |
|---|---|---|---|---|---|
| | Repetiti on | Total | Death count | Mortality (%) | Corrected mortality (%) |
| CK | 1 | 127 | 6 | 4.72 | |
| | 2 | 142 | 11 | 7.75 | |
| | 3 | 103 | 9 | 8.74 | |
| | 4 | 121 | 5 | 4.13 | |
| Average | | 123.25 | 7.75 | 6.34 | |
| 3.125 | 1 | 145 | 24 | 16.55 | 12.42 |
| | 2 | 159 | 38 | 23.90 | 17.51 |
| | 3 | 101 | 26 | 25.74 | 18.63 |
| | 4 | 124 | 20 | 16.13 | 12.52 |
| Average | | 132.25 | 27 | 20.58 | 15.27 |
| 6.25 | 1 | 126 | 36 | 28.57 | 25.03 |
| | 2 | 157 | 60 | 38.22 | 33.03 |
| | 3 | 109 | 39 | 35.78 | 29.63 |
| | 4 | 130 | 58 | 44.62 | 42.23 |
| Average | | 130.5 | 48.25 | 36.80 | 32.48 |
| 12.5 | 1 | 134 | 80 | 59.70 | 57.70 |
| | 2 | 127 | 64 | 50.39 | 46.22 |
| | 3 | 149 | 69 | 46.31 | 41.17 |
| | 4 | 116 | 74 | 63.79 | 62.23 |
| Average | | 131.5 | 71.75 | 55.05 | 51.83 |
| 25 | 1 | 106 | 68 | 64.15 | 62.37 |
| | 2 | 121 | 93 | 76.86 | 74.92 |
| | 3 | 165 | 116 | 70.30 | 67.46 |
| | 4 | 138 | 91 | 65.94 | 64.47 |
| Average | | 132.5 | 92 | 69.31 | 67.30 |

(continued)

| Fosthiazate | | | 24 h | | |
|---|---|---|---|---|---|
| | Repetiti on | Total | Death count | Mortality (%) | Corrected mortality (%) |
| 50 | 1 | 102 | 84 | 82.35 | 81.48 |
| | 2 | 163 | 132 | 80.98 | 79.38 |
| | 3 | 118 | 94 | 79.66 | 77.71 |
| | 4 | 139 | 105 | 75.54 | 74.49 |
| Average | | 130.5 | 103.75 | 79.63 | 78.26 |
| 100 | 1 | 146 | 117 | 80.14 | 79.16 |
| | 2 | 127 | 98 | 77.17 | 75.25 |
| | 3 | 161 | 141 | 87.58 | 86.39 |
| | 4 | 114 | 92 | 80.70 | 79.87 |
| Average | | 137 | 112 | 81.40 | 80.17 |

**Table 13 Toxicity test data for control agent sulfoxamyl against *Meloidogyne incognita***

| Sulfoxamyl | | | 24 h | | | 48 h | | | 72 h | | |
|---|---|---|---|---|---|---|---|---|---|---|---|
| | Repetition | Total | Deat h count | Mort ality (%) | Corre ction Mort ality (%) | Deat h count | Mort ality (%) | Corre ction Mort ality (%) | Deat h count | Mort ality (%) | Correctio n Mortality (%) |
| C K | 1 | 131 | 1 | 0.76 | | 1 | 0.76 | | 3 | 2.29 | |
| | 2 | 118 | 2 | 1.69 | | 3 | 2.54 | | 3 | 2.54 | |
| | 3 | 125 | 1 | 0.80 | | 1 | 0.80 | | 1 | 0.80 | |
| | 4 | 114 | 2 | 1.75 | | 2 | 1.75 | | 2 | 1.75 | |
| Average | | 122 | 1.5 | 1.23 | | 2 | 1.43 | | 2 | 1.84 | |
| 3.1 | 1 | 104 | 15 | 14.42 | 13.36 | 21 | 20.19 | 19.03 | 39 | 37.50 | 36.33 |
| | 2 | 110 | 19 | 17.27 | 16.24 | 30 | 27.27 | 26.21 | 41 | 37.27 | 36.09 |
| | 3 | 127 | 35 | 27.56 | 26.66 | 40 | 31.50 | 30.50 | 52 | 40.94 | 39.84 |
| | 4 | 117 | 21 | 17.95 | 16.93 | 23 | 19.66 | 18.49 | 38 | 32.48 | 31.21 |
| Average | | 114.5 | 22.5 | 19.65 | 18.30 | 29 | 24.89 | 23.56 | 43 | 37.12 | 35.87 |
| 6.2 5 | 1 | 116 | 19 | 16.38 | 15.34 | 36 | 31.03 | 30.03 | 41 | 35.34 | 34.13 |
| | 2 | 108 | 25 | 23.15 | 22.19 | 34 | 31.48 | 30.48 | 36 | 33.33 | 32.08 |
| | 3 | 121 | 20 | 16.53 | 15.49 | 29 | 23.97 | 22.86 | 35 | 28.93 | 27.59 |
| | 4 | 106 | 31 | 29.25 | 28.36 | 37 | 34.91 | 33.96 | 42 | 39.62 | 38.49 |
| Average | | 112.8 | 23.75 | 21.06 | 20.35 | 34 | 30.16 | 29.33 | 39 | 34.15 | 33.07 |
| 12. 5 | 1 | 109 | 56 | 51.38 | 50.77 | 67 | 61.47 | 60.91 | 71 | 65.14 | 64.48 |
| | 2 | 121 | 54 | 44.63 | 43.94 | 69 | 57.02 | 56.40 | 75 | 61.98 | 61.27 |
| | 3 | 125 | 62 | 49.60 | 48.97 | 71 | 56.80 | 56.17 | 85 | 68.00 | 67.40 |
| | 4 | 105 | 53 | 50.48 | 49.86 | 61 | 58.10 | 57.49 | 71 | 67.62 | 67.01 |
| Average | | 115 | 56.25 | 48.91 | 48.39 | 67 | 58.26 | 57.74 | 76 | 65.65 | 65.04 |

(continued)

| Sulfoxamyl | | | 24 h | | | 48 h | | | 72 h | | |
|---|---|---|---|---|---|---|---|---|---|---|---|
| | Repetition | Total | Deat h count | Mort ality (%) | Corre ction Mort ality (%) | Deat h count | Mort ality (%) | Corre ction Mort ality (%) | Deat h count | Mort ality (%) | Correctio n Mortality (%) |
| 25 | 1 | 118 | 78 | 66.10 | 65.68 | 78 | 66.10 | 65.61 | 78 | 66.10 | 65.46 |
| | 2 | 127 | 75 | 59.06 | 58.55 | 81 | 63.78 | 63.25 | 95 | 74.80 | 74.33 |
| | 3 | 106 | 68 | 64.15 | 63.70 | 72 | 67.92 | 67.46 | 90 | 84.91 | 84.62 |
| | 4 | 130 | 90 | 69.23 | 68.85 | 90 | 69.23 | 68.78 | 90 | 69.23 | 68.65 |
| Average | | 120.3 | 77.75 | 64.66 | 64.19 | 80 | 66.74 | 66.28 | 88 | 73.39 | 73.27 |
| 50 | 1 | 117 | 101 | 86.32 | 86.15 | 108 | 92.31 | 92.20 | 115 | 98.29 | 98.26 |
| | 2 | 107 | 88 | 82.24 | 82.02 | 95 | 88.79 | 88.62 | 99 | 92.52 | 92.38 |
| | 3 | 122 | 101 | 82.79 | 82.57 | 111 | 90.98 | 90.85 | 120 | 98.36 | 98.33 |
| | 4 | 102 | 92 | 90.20 | 90.07 | 98 | 96.08 | 96.02 | 101 | 99.02 | 99.00 |
| Average | | 112 | 95.5 | 85.27 | 85.21 | 103 | 91.96 | 91.92 | 109 | 97.10 | 96.99 |
| 10 0 | 1 | 109 | 105 | 96.33 | 96.28 | 108 | 99.08 | 99.07 | 109 | 100.0 0 | 100.00 |
| | 2 | 102 | 102 | 100.0 0 | 100.0 0 | 102 | 100.0 0 | 100.0 0 | 102 | 100.0 0 | 100.00 |
| | 3 | 121 | 120 | 99.17 | 99.16 | 121 | 100.0 0 | 100.0 0 | 121 | 100.0 0 | 100.00 |
| | 4 | 108 | 107 | 99.07 | 99.06 | 108 | 100.0 0 | 100.0 0 | 108 | 100.0 0 | 100.00 |
| Average | | 110 | 108.5 | 98.64 | 98.63 | 110 | 99.77 | 99.77 | 110 | 100.0 0 | 100.00 |

**Table 14 Determination data of inhibitory effect for sulfoxamyl and oxamyl on hatching of *Meloidogyne incognita* eggs**

| Agent | Concentration (mg/L) | Total number of eggs | Number of hatched eggs/7 d | Number of unhatched eggs/7 d |
|---|---|---|---|---|
| CK | --- | 134 | 113 | 21 |
| | | 136 | 116 | 20 |
| | | 129 | 113 | 16 |
| Sulfoxamyl | 100 | 137 | 44 | 93 |
| | | 125 | 40 | 85 |
| | | 130 | 42 | 88 |
| | 50 | 138 | 57 | 81 |
| | | 130 | 55 | 75 |
| | | 126 | 49 | 77 |
| | 25 | 130 | 85 | 45 |
| | | 120 | 79 | 41 |
| | | 124 | 86 | 38 |
| | 12.5 | 115 | 83 | 32 |

(continued)

| Agent | Concentration (mg/L) | Total number of eggs | Number of hatched eggs/7 d | Number of unhatched eggs/7 d |
|---|---|---|---|---|
| | | 118 | 85 | 33 |
| | | 128 | 88 | 40 |
| | 6.25 | 133 | 102 | 31 |
| | | 117 | 92 | 25 |
| | | 129 | 96 | 33 |
| Oxamyl | 400 | 125 | 12 | 113 |
| | | 132 | 15 | 117 |
| | | 132 | 11 | 121 |
| | 200 | 123 | 26 | 97 |
| | | 121 | 20 | 101 |
| | | 126 | 23 | 103 |
| | 100 | 130 | 62 | 68 |
| | | 132 | 72 | 60 |
| | | 129 | 71 | 58 |
| | 50 | 136 | 83 | 53 |
| | | 139 | 86 | 53 |
| | | 130 | 78 | 52 |
| | 25 | 128 | 81 | 47 |
| | | 128 | 92 | 36 |
| | | 132 | 90 | 42 |
| Conclusion: indoor efficacy tests show that compared with existing products such as oxamyl and fosthiazate, sulfoxamyl has a higher killing activity against *Meloidogyne incognita* J2, and also has a better inhibitory effect on nematode eggs. | | | | |

Examples for preparing sulfoxamyl by using oxamyl as a raw material are shown in Examples 1 to 11:

## Example 1

21.9 g (0.1 mol) of oxamyl and 50 mL of acetic acid were added into a 100 mL four-necked flask equipped with a stirrer and a thermometer, stirred to dissolve, added with 6 g (0.06 mol) of 98% sulfuric acid at room temperature, slowly added with 31.5 g (0.25 mol) of 27% hydrogen peroxide, stirred at room temperature for 10 min, then kept at 35 °C for about 3 h to precipitate crystals, and the reaction was completed in about 5 h. After cooling to 0 °C, a resulting cooled material was filtered and separated to obtain white crystals, which were dried at 60 °C to obtain 19.8 g with a content of 98.1%. The content in the mother liquor is 4.9%, equivalent to 4.1 g. The total equivalent amount of the product is 23.5 g, and the total yield is 93.6%.

## Example 2

The mother liquor after separation from the product of Example 1 was used as a reaction medium, 21.9 g (0.1 mol) of oxamyl was added and dissolved under stirring, 31.5 g (0.25 mol) of 27% hydrogen peroxide was slowly added and stirred at room temperature for 10 min, then kept at 35 °C for about 5 h to precipitate crystals, and the reaction was completed in about 6 h. After cooling to 0 °C, a resulting cooled material was filtered and separated to obtain white crystals, which were dried at 60 °C to obtain 24.8 g with a content of 98.5%, and a yield of 97.2%.

This batch of mother liquor could be reused once more without adding acetic acid and catalyst sulfuric acid.

In Examples 3 to 11, certain changes were made to the process conditions in Example 1 to illustrate the effects of

factors such as reaction medium, catalyst, and hydrogen peroxide on the reaction rate and yield. The operating processes were the same as those in Example 1 and are not described in detail here. The test conditions and results of each batch are shown in Table 15.

**Table 15 Influence of process conditions for preparing sulfoxamyl on yield**

| SN | Solvent/dose/ mL | Catalyst/mol | Hydrogen peroxide/m ol | Precipitati on time/h | Reaction time/h | Total yield/% |
|---|---|---|---|---|---|---|
| Example 1 | Acetic acid/50 | Sulfuric acid/0.06 | 0.25 | 3 | 5 | 93.6 |
| Example 2 | Reusing the mother liquor of Example 2 | | 0.25 | 5 | 6 | 98.8 |
| Example 3 | 75% acetic acid/50 | Sulfuric acid/0.06 | 0.25 | 4 | 6 | 98.5 |
| Example 4 | 50% acetic acid/50 | Sulfuric acid/0.06 | 0.25 | 5 | 6 | 97.2 |
| Example 5 | Formic acid/50 | Sulfuric acid/0.06 | 0.25 | Unprecipit ated | 1 | 97.6 |
| Example 6 | Propionic acid/50 | Sulfuric acid/0.06 | 0.25 | 3 | 6 | 90.8 |
| Example 7 | n-Butyric acid/50 | Sulfuric acid/0.06 | 0.25 | 5 | 10 | 85.6 |
| Example 8 | Acetic acid/50 | Sulfuric acid/0 | 0.25 | 136 | 168 | 48.6 |
| Example 9 | Acetic acid/50 | Hydrochloric acid/0.06 | 0.25 | 5 | 8 | 96.0 |
| Example 10 | Acetic acid/50 | Trifluoroaceti c acid/0.06 | 0.25 | 5 | 10 | 92.3 |
| Example 11 | Acetic acid/50 | Sulfuric acid/0.06 | 0.30 | 3 | 4 | 97.7 |
| NOTE: the input amount of the raw material oxamyl in Examples 1 to 11 was 0.1 mol, with a purity of 97% (w/w). | | | | | | |

Examples for preparing sulfoxamyl oxime by using oxamyl oxime are shown in Examples 12 to 25:

### Example 12

16.2 g (0.1 mol) of oxamyl oxime and 50 mL of acetic acid were added into a 100 mL four-necked flask equipped with a stirrer and a thermometer, added with 6 g (0.06 mol) of 98% sulfuric acid under stirring at room temperature, slowly added with 31.5 g (0.25 mol) of 27% hydrogen peroxide, stirred at room temperature for 10 min, then kept at 35 °C for about 3 h to precipitate crystals, and the reaction was completed in about 5 h. After cooling to 0 °C, a resulting cooled material was filtered and separated to obtain white crystals, which were dried at 60 °C to obtain 16.5 g with a content of 98.5%. The content in the mother liquor is 3.3%, equivalent to 2.6 g. The total equivalent amount of the product is 18.9 g, and the total yield is 97.4%.

### Example 13

The mother liquor after separation from the product of Example 12 was used as a reaction medium, 16.2 g (0.1 mol) of oxamyl oxime was added and dissolved under stirring, 31.5 g (0.25 mol) of 27% hydrogen peroxide was slowly added and stirred at room temperature for 10 min, then kept at 35 °C for about 4 h to precipitate crystals, and the reaction was completed in about 10 h. After cooling to 0 °C, a resulting cooled material was filtered and separated to obtain white crystals, which were dried at 60 °C to obtain 19.5 g with a content of 98%, and a yield of 98.5%. This batch of mother liquor could be reused once more without adding acetic acid and catalyst sulfuric acid.

A resulting sulfoxamyl oxime was mixed with MIC and condensed to obtain the sulfoxamyl. Alternatively, the resulting sulfoxamyl oxime, MCC, and an acid-binding agent were mixed and subjected a resulting mixture to condensation to obtain sulfoxamyl.

In Examples 14 to 25, certain changes were made to the process conditions in Example 12 to illustrate the effects of factors such as reaction medium, catalyst, hydrogen peroxide, and reaction temperature on the oxidation reaction rate and yield of sulfoxamyl oxime. The operating processes were the same as those in Example 12 and are not described in detail here. The test conditions and results of each batch are shown in Table 16.

**Table 16 Influence of process conditions for preparing sulfoxamyl oxime on yield**

| SN | Solvent/dos e/mL | Catalyst/mol | Hydrogen peroxide/ mol | Reaction temperatur e (°C) | Precipitati on time/h | Reaction time/h | Total yield/% |
|---|---|---|---|---|---|---|---|
| Example 12 | Acetic acid/50 | Sulfuric acid/0.06 | 0.25 | 35 | 3 | 5 | 97.4 |
| Example 13 | Reusing the mother liquor of Example 12 | | 0.25 | 35 | 4 | 10 | 98.5 |
| Example 14 | 75% acetic acid/50 | Sulfuric acid/0.06 | 0.25 | 35 | 4 | 10 | 97.2 |
| Example 15 | 50% acetic acid/50 | Sulfuric acid/0.06 | 0.25 | 35 | 8 | 18 | 97.2 |
| Example 16 | Formic acid/50 | Sulfuric acid/0.06 | 0.25 | 35 | 0.5 | 1 | 97.8 |
| Example 17 | Propionic acid/50 | Sulfuric acid/0.06 | 0.25 | 35 | 3 | 6 | 89.8 |
| Example 18 | *n*-Butyric acid/50 | Sulfuric acid/0.06 | 0.25 | 35 | 5 | 8 | 84.6 |
| Example 19 | Acetic acid/50 | Sulfuric acid/0 | 0.25 | 35 | 144 | 168 | 32.5 |
| Example 20 | Acetic acid/50 | Hydrochlori c acid/0.06 | 0.25 | 35 | 3 | 5 | 97.3 |
| Example 21 | Acetic acid/50 | Trifluoroacet ic acid/0.06 | 0.25 | 35 | 4 | 5 | 93.6 |
| Example 22 | Acetic acid/50 | Sulfuric acid/0.06 | 0.30 | 35 | 3 | 4 | 98.1 |
| Example 23 | Acetic acid/50 | Sulfuric acid/0.06 | 0.25 | 50 | 0.5 | 3 | 97.1 |
| Example 24 | Acetic acid/50 | Sulfuric acid/0.06 | 0.25 | 65 | Unprecipi tated | 0.5 | 96.5 |
| Example 25 | Acetic acid/50 | Sulfuric acid/0.06 | 0.25 | 80 | Unprecipi tated | 0.5 | 95.6 |
| NOTE: the input amount of the raw material oxamyl oxime in Examples 12 to 25 was 0.1 mol, with a purity of 98% (w/w); the total yield refers to the yield of sulfoxamyl oxime. | | | | | | | |

The above are merely preferred embodiments of the present disclosure. It should be noted that a person of ordinary skill in the art may further make several improvements and modifications without departing from the principle of the present disclosure, but such improvements and modifications should be deemed as falling within the scope of the present disclosure.

## Claims

1. Use of sulfoxamyl in killing nematodes.

2. The use of claim 1, wherein the sulfoxamyl is used in a form of a composition, and a dosage form of the composition is

at least one selected from the group consisting of a granule, a powder, a wettable powder, an emulsifiable concentrate, a suspension concentrate, a dry suspension, a concentrated emulsion, and a sustained-release agent.

3. The use of claim 2, wherein the composition further comprises at least one selected from the group consisting of an auxiliary agent, a carrier, and a solvent.

4. The use of claim 3, wherein the auxiliary agent comprises at least one selected from the group consisting of a binder and a stabilizer; wherein the binder comprises at least one selected from the group consisting of paraffin and polyvinyl alcohol (PVA); and the stabilizer comprises at least one selected from the group consisting of phosphoric acid, oxalic acid, tartaric acid, and citric acid.

5. The use of claim 3, wherein the carrier comprises at least one selected from the group consisting of quartz sand, attapulgite, and a clay particle.

6. The use of claim 3, wherein the solvent comprises at least one selected from the group consisting of water, an alcohol, an ester, an aromatic hydrocarbon, and an ether.

7. The use of claim 2, wherein a method for field application of the composition is selected from the group consisting of hole application, furrow application, broadcast application, blending with a toxic soil, and root irrigation.

8. An oxidation system for oxidizing a sulfur group in oxamyl or oxamyl oxime into a sulfone group, comprising an oxidant, a catalyst, and a reaction medium; wherein

   the oxidant is hydrogen peroxide;
   the catalyst is a strong acid; and
   the reaction medium is selected from the group consisting of a carboxylic acid and an aqueous solution of the carboxylic acid.

9. The oxidation system of claim 8, wherein the hydrogen peroxide has a mass concentration not greater than 75%.

10. The oxidation system of claim 8, wherein the strong acid is selected from the group consisting of an inorganic strong acid and an organic strong acid;

   wherein the inorganic strong acid is at least one selected from the group consisting of sulfuric acid, hydrochloric acid, nitric acid, perchloric acid, hydrobromic acid, hydroiodic acid, and phosphoric acid; and
   the organic strong acid is selected from the group consisting of a halogenated carboxylic acid and a sulfonic acid; wherein the halogenated carboxylic acid is at least one selected from the group consisting of trifluoroacetic acid, trifluoropropionic acid, trifluorobutyric acid, monochloroacetic acid, dichloroacetic acid, and trichloroacetic acid; and
   the sulfonic acid is selected from the group consisting of benzenesulfonic acid and alkylbenzenesulfonic acid; and an alkyl group in the alkylbenzenesulfonic acid has a carbon number of 1 to 12.

11. The oxidation system of claim 8, wherein the carboxylic acid comprises at least one selected from the group consisting of formic acid, acetic acid, propionic acid, and butyric acid; and the aqueous solution of the carboxylic acid comprises at least one selected from the group consisting of an aqueous solution of the formic acid, an aqueous solution of the acetic acid, an aqueous solution of the propionic acid, and an aqueous solution of the butyric acid.

12. The oxidation system of claim 8 or 11, wherein the aqueous solution of the carboxylic acid has a mass concentration not less than 50%.

13. The oxidation system of claim 8, wherein the reaction medium is at least one selected from the group consisting of formic acid, an aqueous solution of the formic acid, acetic acid, an aqueous solution of the acetic acid, propionic acid, an aqueous solution of the propionic acid, butyric acid, and an aqueous solution of the butyric acid.

14. A method for preparing sulfoxamyl, comprising:
subjecting oxamyl to oxidation in the presence of the oxidation system of any one of claims 8 to 13 to obtain the sulfoxamyl.

**15.** The method of claim 14, wherein a molar amount of the oxidant is not less than 2 times a molar amount of the oxamyl based on the hydrogen peroxide as an active ingredient;

a molar amount of the catalyst is not less than 0.01 times a molar amount of the oxamyl based on the strong acid as an active ingredient;
a mass of the reaction medium is not less than 2 times a mass of the oxamyl based on the carboxylic acid as an active ingredient; and
the oxidation is conducted at a temperature of 0 °C to 100 °C for 1 h to 24 h.

**16.** The method of claim 14 or 15, wherein after the oxidation is completed, the method further comprises: cooling a resulting oxidation system to 0 °C, subjecting a resulting cooled product to solid-liquid separation to obtain a solid, and then drying the solid.

**17.** A method for preparing sulfoxamyl, comprising:

subjecting oxamyl oxime to oxidation in the presence of the oxidation system of any one of claims 8 to 13 to obtain sulfoxamyl oxime; and
subjecting the sulfoxamyl oxime and a monomer to condensation to obtain the sulfoxamyl;
wherein the monomer is selected from the group consisting of methyl isocyanate (MIC) and carbamoyl chloride (MCC).

**18.** The method of claim 17, wherein a molar amount of the oxidant is not less than 2 times a molar amount of the oxamyl oxime based on the hydrogen peroxide as an active ingredient;

a molar amount of the catalyst is not less than 0.01 times a molar amount of the oxamyl oxime based on the strong acid as an active ingredient;
a mass of the reaction medium is not less than 2 times a mass of the oxamyl oxime based on the carboxylic acid as an active ingredient; and
the oxidation is conducted at a temperature of 0 °C to 100 °C for 1 h to 24 h.

**19.** The method of claim 17 or 18, wherein after the oxidation is completed, the method further comprises: cooling a resulting oxidation system to 0 °C, subjecting a resulting cooled product to solid-liquid separation to obtain a solid, and then drying the solid.

**20.** The method of claim 17, wherein under a condition that the monomer is the MIC, a molar ratio of the sulfoxamyl oxime to the MIC is in a range of 1:1.0 to 1:1.5.

**21.** The method of claim 17, wherein under a condition that the monomer is the MCC, the method further comprises adding an acid-binding agent; wherein

the acid-binding agent comprises an organic tertiary amine; and the organic tertiary amine comprises at least one selected from the group consisting of triethylamine (TEA) and pyridine; and
a molar ratio of the sulfoxamyl oxime, the MCC, and the acid-binding agent is in a range of 1:(1.0-1.8):(1.0-1.8).

**22.** The method of claim 17, 20, or 21, wherein the condensation is conducted at a temperature of 10 °C to 80 °C for 0.5 h to 2 h.

## INTERNATIONAL SEARCH REPORT

International application No.
**PCT/CN2024/103087**

**A. CLASSIFICATION OF SUBJECT MATTER**

A01N 47/24(2006.01)i; A01P 5/00(2006.01)i; C07C 315/02(2006.01)i; C07C 315/04(2006.01)i; C07C 317/44(2006.01)i

According to International Patent Classification (IPC) or to both national classification and IPC

**B. FIELDS SEARCHED**

Minimum documentation searched (classification system followed by classification symbols)

A01N A01P C07C

Documentation searched other than minimum documentation to the extent that such documents are included in the fields searched

Electronic data base consulted during the international search (name of data base and, where practicable, search terms used)

ENTXTC; CNTXT; DWPI; WOTXT; USTXT; EPTXT; CNKI; 万方, WANFANG; ISI Web of Science; STN: 宁夏金海宏昇化工, 郝立勇, 郭传垒, 徐坤, 殷翠平, 氧线威, 线虫, 杀线威, 氧化, 催化, 酸, 式I, 式II, 34763-86-5, 7722-84-1, oxycarb, nematode, preparat+, oxidat+, Catalyst, Oxidizi+, Hydrogen peroxide, H2O2, acid

**C. DOCUMENTS CONSIDERED TO BE RELEVANT**

| Category* | Citation of document, with indication, where appropriate, of the relevant passages | Relevant to claim No. |
|---|---|---|
| PX | CN 117770270 A (NINGXIA JINHAI HONGSHENG CHEMICAL CO., LTD.) 29 March 2024 (2024-03-29)<br>entire document | 1-22 |
| A | DE 2119700 A1 (E. I. DU PONT DE NEMOURS AND COMPANY) 23 December 1971 (1971-12-23)<br>claims 1, 11-13, and 15, and description, page 6 | 1-22 |
| A | HARVEY, J. Jr. et al. "Metabolism of Oxamyl in Plants"<br>*Journal of Agricultural and Food Chemistry,* Vol. 26, No. 3, 01 May 1978 (1978-05-01), 529-36<br>ISSN: 0021-8561,<br>entire document | 1-22 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **26 July 2024** | **13 August 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**
**Information on patent family members**

International application No.
**PCT/CN2024/103087**

| Patent document cited in search report | | | Publication date (day/month/year) | Patent family member(s) | | | Publication date (day/month/year) |
|---|---|---|---|---|---|---|---|
| CN | 117770270 | A | 29 March 2024 | None | | | |
| DE | 2119700 | A1 | 23 December 1971 | US | 3819700 | A | 25 June 1974 |
| | | | | CA | 924304 | A | 10 April 1973 |
| | | | | CH | 555816 | A | 15 November 1974 |
| | | | | FR | 2090645 | A5 | 14 January 1972 |
| | | | | NL | 7105463 | A | 26 October 1971 |
| | | | | ES | 390424 | A1 | 01 July 1974 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202311774550
- DE 2119700